# EUROPÄISCHE PATENTANMELDUNG

(11) **EP 0 539 987 A2**
(43) Veröffentlichungstag der Anmeldung: **05.05.1993**
(21) Anmeldenummer: 92118513.8
(22) Anmeldetag: 29.10.1992
(51) Int. Cl.: C07D 475/04

(54) **Verfahren zur Reinigung von rohen Erdalkalimetallsalzen von N(5)-Methyl-5,6,7,8-tetrahydrofolsäure**

(30) Priorität: 29.10.1991 CH 3153/91
(71) Anmelder: Cerbios-Pharma S.A., CH-6917 Barbengo (CH)
(72) Erfinder: Attilio, Melera, CH-6926 Montagnola (CH); Fabrizio, Marazza, CH-6986 Novaggio (CH)
(74) Vertreter: Zink-Wild, Markus Peter

(57) **Zusammenfassung**

Das erfindungsgemässe Verfahren zur Reinigung - insbesondere zur Entfernung von Bor enthaltenden Verbindungen - von rohen Erdalkalimetallsalzen von N(5)-Methyl-5,6,7,8-tetrahydrofolsäure der Formel I in der Form des (6RS)-Diastereoisomerengemisches oder in der Form der einzelnen (6S)- oder (6R)-Diastereoisomeren, erhalten aus der Reduktion eines wasserlöslichen Alkalimetall- oder Ammoniumsalzes von N(5),N(10)-Methylen-5,6,7,8-tetrahydrofolsäure der Formel II in der Form des (6RS)-Diastereoisomerengemisches oder in der Form der einzelnen (6S)- oder (6R)-Diastereoisomeren in wässriger Lösung mit wenigstens einem Alkalimetallborhydrid, gefolgt von der Hinzugabe wenigstens eines Aequivalentes eines Erdalkalimetallsalzes und der Abtrennung des entstandenen Festkörpers, welcher die genannten Salze der Formel I enthält, ist dadurch gekennzeichnet, dass man den genannten Festkörper in Wasser löst und dazu wenigstens eine Verbindung A hinzugibt, welche insbesondere für die Komplexierung von Bor enthaltenden Verbindungen B geeignet ist, dann das so hergestellte Gemisch C einengt, wobei eine Kristallisation eintritt, und die entstandenen, reinen Kristalle der Formel I mittels Filtration oder Zentrifugation isoliert.

## Beschreibung

Die vorliegende Erfindung betrifft ein Verfahren zur Reinigung von rohen Erdalkalimetallsalzen von N(5)-Methyl-5,6,7,8-tetrahydrofolsäure.

5,6,7,8-Tetrahydrofolsäure 2 - abgekürzt mit THF - kann mittels Reduktion aus Folsäure 1 hergestellt werden. Die üblichen Methoden sind:
1. die katalytische Hydrierung (siehe z.B. F.M. Huennekens, P.P.K.Ho und K.G. Scrimgeour, Methods in Enzymology, (1963) 6, 802),
2. die Natriumborhydrid Reduktion von 7,8-Dihydrofolsäure 3, erhalten aus der Natriumdithionit Reduktion des Na-Salzes von Folsäure 1 (E.K. Kalifa, A.N. Ganguly, J.H. Bieri und M. Viscontini, Helv. Chim. Acta, (1980), 63,2554; K.G. Scrimgeour, Methods in Enzymology, (1980), 66,517),
3. die direkte Reduktion von Folsäure 1 mit Natriumborhydrid (z.B.: J.A. Blair, K.J. Saunders Anal. Bioch. (1970), 34,376). Siehe Schema 1.

Die Methylierung von THF 2 zur N(5)-Methyl-THF der Formel I wird üblicherweise mittels Reaktion von THF 2 mit Formaldehyd und nachträglicher Reduktion des vorzugsweise in situ gebildeten N⁵,N¹⁰-Methylen-THF 5 mit Natriumborhydrid durchgeführt; siehe K.O. Donaldson und Keresztesy J.C., J. Biol. Chem., (1962), 237,3815; und V.S. Gupta und F.M. Huennekens, Arch. Biochem. Biophys. (1967), 120,712. Siehe Schema 1.

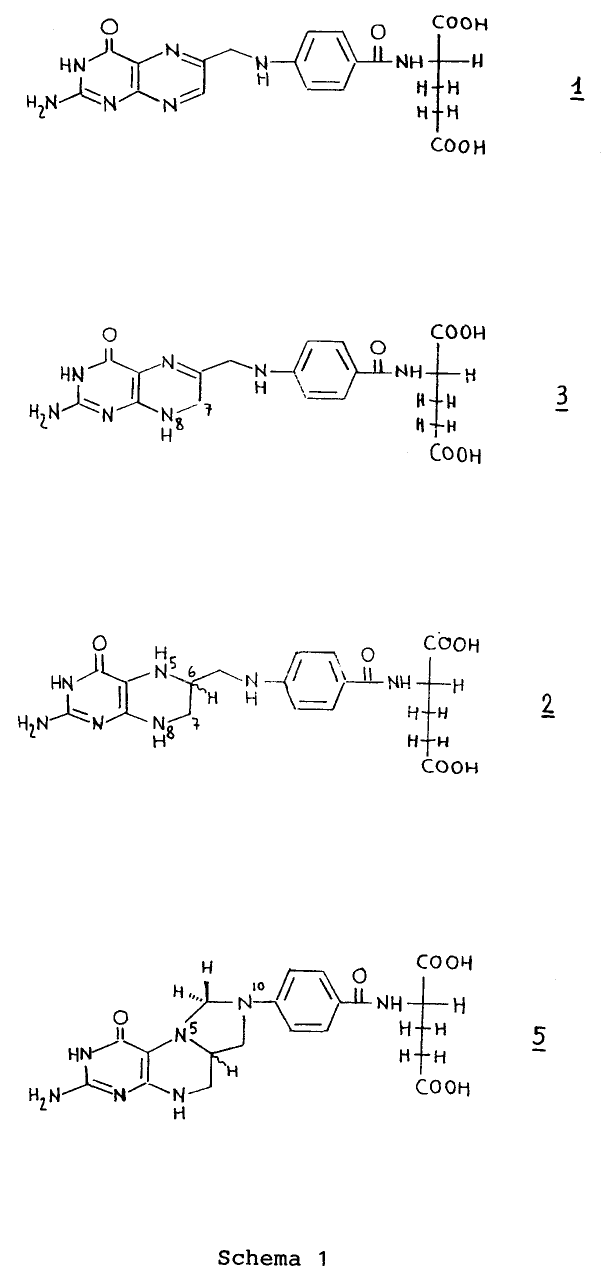

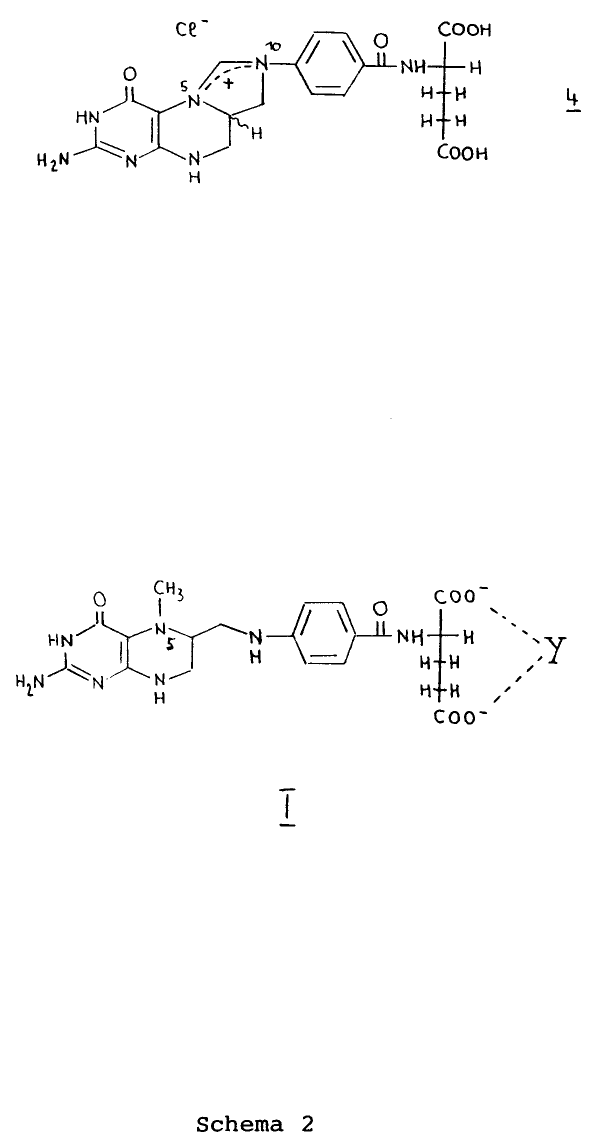

Die Isolierung der reinen Verbindungen der Formel I, beispielsweise N(5)-Methyl-THF.Ca²⁺, aus dem Gemisch der Reaktionsprodukte ist wegen der Anwesenheit von relativ grossen Mengen an Bor enthaltenden Verbindungen problematisch.

Zwei Methoden zur Umgehung dieser Schwierigkeit wurden publiziert:
1. Adsorption/Desorption des Produktes an Aktivkohle (F. Gennari, Bioresearch SpA, CH PS 635 344). Hierin wird eine aufwendige und mit beträchtlichen Produkteverlusten verbundene Methode beschrieben, wobei die Ausbeute höchstens 50% erreichen kann.
2. Gemäss der CH PS 649 550 werden die Verbindungen der Formel I mittels NaBH₄-Reduktion von N(5),N(10)-Methenyl-THF.Cl⁻ 4 über das intermediär auftretende N(5),N(10)-Methylen-THF 5 hergestellt. Siehe Schema 2. Dabei werden die Bor enthaltenden Verbindungen mit einer Polyhydroxy-Verbindung entfernt, wobei Sorbit bevorzugt ist. Dabei wird die Polyhydroxy-Verbindung in einer drei- bis sechsfachen Gewichtsmenge, bezogen auf den N(5)-Methyl-THF.Ca²⁺ Gehalt, verwendet. Der Nachteil dieses Verfahrens besteht in der Verwendung eines grossen Ueberschusses an Reagens (Polyhydroxy-Verbindung).

Es ist ein Ziel der vorliegenden Erfindung, ein neues, alternatives, effizientes, einfaches, wirtschaftliches und in grosstechnischem Massstab durchführbares Verfahren zur Reinigung - insbesondere zur Entfernung von Bor enthaltenden Verbindungen - von rohen Erdalkalimetallsalzen von N(5)-Methyl-5,6,7,8-THF zur Verfügung zu stellen.

Völlig überraschend wurde nun gefunden, dass sich dieses Problem leicht lösen lässt, indem man die zu reinigende wässrige Lösung von N(5)-Methyl-THF.Ca²⁺ mit einem Ethylendiamintetraessigsäure-Salz oder einem analogen Komplexierungsmittel, wie Nitrilotriessigsäure oder einem polymergebundenen Komplexierungsmittel, wie Chelosolve^{R}, versetzt und die erhaltene Lösung zur Kristallisation bringt.

Das erfindungsgemässe Verfahren zur Reinigung - insbesondere zur Entfernung von Bor enthaltenden Verbindungen - von rohen Erdalkalimetallsalzen von N(5)-Methyl-5,6,7,8-tetrahydrofolsäure der Formel I 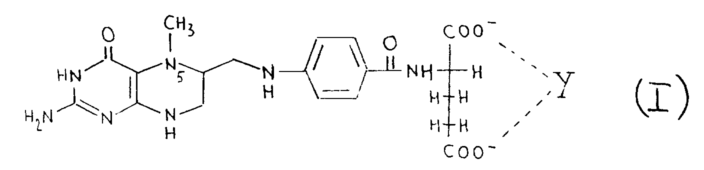
in der Form des (6RS)-Diastereoisomerengemisches oder in der Form der einzelnen (6S)- oder (6R)-Diastereoisomeren,
worin
Y ein Erdalkalimetallkation, insbesondere Ca²⁺ oder Mg²⁺, bedeutet,
erhalten aus der Reduktion eines wasserlöslichen Alkalimetall- oder Ammoniumsalzes von N(5),N(10)-Methylen-5,6,7,8-tetrahydrofolsäure der Formel II 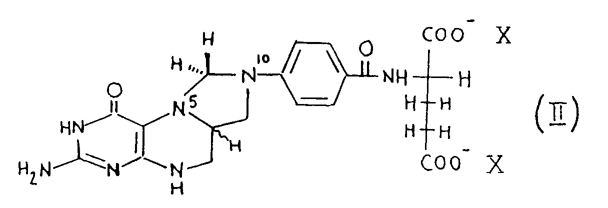
in der Form des (6RS)-Diastereoisomerengemisches oder in der Form der einzelnen (6S)- oder (6R)-Diastereoisomeren,
worin
X ein Alkalimetallkation oder ein Ammoniumion bedeutet,
in wässriger Lösung mit wenigstens einem Alkalimetallborhydrid,
gefolgt von der Hinzugabe wenigstens eines Aequivalentes eines Erdalkalimetallsalzes und der Abtrennung des entstandenen Festkörpers, welcher die genannten Salze der Formel I enthält,
ist dadurch gekennzeichnet, dass man den genannten Festkörper in Wasser löst und dazu wenigstens eine Verbindung A hinzugibt, welche insbesondere für die Komplexierung von Bor enthaltenden Verbindungen B geeignet ist,
dann das so hergestellte Gemisch C einengt, wobei eine Kristallisation eintritt, und die entstandenen, reinen Kristalle der Formel I mittels Filtration oder Zentrifugation isoliert.

Bevorzugte Ausführungsformen dieses Verfahrens sind in den abhängigen Ansprüchen definiert.

Es ist bevorzugt, mit dem erfindungsgemässen Verfahren die Verbindung N(5)-Methyl-THF.Ca²⁺ zu reinigen.

Diese Verbindung, wie auch alle übrigen Verbindungen der Formel I, kann in der Form des (6RS)-Diastereoisomerengemisches oder in der Form der einzelnen (6S)- oder (6R)-Diastereoisomeren gereinigt werden. Die entsprechende Diastereoisomerentrennung ist in der Schweizerischen Patentanmeldung Nr. 01 255/90-1 sowie in der entsprechenden europäischen Patentanmeldung Nr. 91 105 715.6 mit der Veröffentlichungs Nr. 0 455 013 beschrieben.

Im zu reinigenden Rohprodukt können verschiedene Bor enthaltende Verbindungen B enthalten sein, wie etwa Tetraborat, Metaborat, etc., siehe Karl A. Hofmann, Anorganische Chemie, 19. Auflage, 1966, Vieweg Verlag, Seite 394 ff.

Im zu reinigenden Rohprodukt wurde gewöhnlich ein Gehalt von mehr als etwa 1000 ppM an Borsäure gemessen; zur Messmethode siehe Ulrike de la Chevallerie-Haaf, Axel Meyer und Günter Henze, Fresenius Z. Anal. Chem. (1986) 323:266-270.

Im gemäss dem erfindungsgemässen Verfahren mit EDTA.2Na⁺ gereinigten Produkt wurde nur noch ein Gehalt von etwa 10 ppM bis etwa 50 ppM an Borsäure gemessen.

Wurde das N(5)-Methyl-THF.Ca²⁺ ohne die Zugabe der Verbindung A aber im übrigen gemäss dem erfindungsgemässen Verfahren umkristallisiert, so wurde ein teilweise gereinigtes Produkt erhalten, bei welchem ein Gehalt von etwa 500 ppM an Borsäure gemessen wurde.

Die Ausbeute an gereinigtem Kristallisationsprodukt betrug etwa 90 %. Das erhaltene, kristalline N(5)-Methyl-THF.Ca²⁺ hatte eine Reinheit von mehr als 99%.

Ein weiterer Vorteil der Kristallisation von N⁵-Methyl-THF.Ca²⁺ in Anwesenheit von einem solchen Komplexierungsmittel ist die Tatsache, dass damit auch Spuren von Schwermetallen entfernt werden, welche bekanntlich die Oxidation und die Zersetzung von N⁵-Methyl-THF katalysieren (J.A. Blair, A.J. Pearson, A.J. Robb, J.Chem. Soc. Perkin II, (1975), 18).

Die gemäss dem erfindungsgemässen Verfahren gereinigten Erdalkalimetallsalze von N(5)-Methyl-5,6,7,8-tetrahydrofolsäure der Formel I können einzeln oder in beliebiger Kombination als wenigstens eine aktive Komponente in einem Arzneimittel für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen verwendet werden. Dabei sind diese Salze in einer Menge von 1 mg bis 500 mg, insbesondere von 5 mg bis 150 mg, pro Dosiseinheit im Arzneimittel vorhanden, wobei das Arzneimittel vorzugsweise in Form eines parenteralen und/oder oralen, pharmazeutischen Präparates vorliegt.

Das nachfolgende Beispiel dient der Illustration der vorliegenden Erfindung.

### Beispiel

Zu einer wässrigen Lösung von 50 g rohem Natrium-(6RS)-N⁵-Methyl-tetrahydrofolat (hergestellt nach: J.A. Blair, K.J. Saunders, Anal. Biochem. (1970), 34,376), gerührt bei Raumtemperatur unter einer Stickstoffatmosphäre, wurden 40 ml einer 30%-igen Calciumchlorid-Lösung zugegeben. Das erhaltene Gemisch wurde dann über Nacht bei einer Temperatur von 5°C weiter gerührt, wobei das rohe Calcium-(6RS)-N⁵-Methyl-tetrahydrofolat auskristallisierte und mittels Filtration isoliert wurde.

Eine HPLC-Analyse dieses Produktes (reverse phase Säule) ergab eine Reinheit von 97.5 %.

Eine Borsäure-Gehaltsbestimmung ergab etwa 1000 ppM Borsäure.

Das so erhaltene Rohprodukt wurde dann bei einer Temperatur von 70°C unter einer Stickstoffatmosphäre in 2 l Wasser gelöst und mit 400 mg Dinatrium-ethylendiamin-tetraessigsäure-dihydrat versetzt. Diese Lösung wurde heiss über eine Schicht Aktivkohle-Celite filtriert und dann unter vermindertem Druck bei einer Temperatur von 45 - 50°C zu ungefähr 1/4 des ursprünglichen Volumens konzentriert. Während des Konzentrierens begann das Calcium-(6RS)-N⁵-Methyl-tetrahydrofolat zu kristallisieren. Die so erhaltene Suspension wurde über Nacht unter einer Stickstoffatmosphäre bei einer Temperatur von 5°C gerührt. Das kristalline Produkt wurde mittels Filtration isoliert, einmal mit deionisiertem Wasser und einmal mit 85%-igem Aethanol gewaschen und unter reduziertem Druck bei einer Temperatur von 70°C getrocknet.

Auf diese Weise wurden 38 g reines Calcium-(6RS)-tetrahydrofolat isoliert.
- HPLC-Analyse (reverse phase Säule):: 99.8 % Reinheit
99.7 % Gehalt
(bzgl. internem SAPEC Standard)
- Borsäure-Gehalt:: etwa 50 ppm.

Das gleiche Verfahren wurde ohne die Zugabe von Aethylendiamintetraessigsäure durchgeführt, wobei ein Produkt erhalten wurde, welches etwa 500 ppM Borsäure enthielt.

## Patentansprüche

1. Verfahren zur Reinigung - insbesondere zur Entfernung von Bor enthaltenden Verbindungen - von rohen Erdalkalimetallsalzen von N(5)-Methyl-5,6,7,8-tetrahydrofolsäure der Formel I 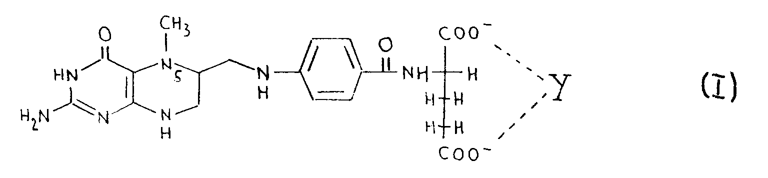 in der Form des (6RS)-Diastereoisomerengemisches oder in der Form der einzelnen (6S)- oder (6R)-Diastereoisomeren,
worin
Y ein Erdalkalimetallkation, insbesondere Ca²⁺ oder Mg²⁺, bedeutet,
erhalten aus der Reduktion eines wasserlöslichen Alkalimetall- oder Ammoniumsalzes von N(5),N(10)-Methylen-5,6,7,8-tetrahydrofolsäure der Formel II 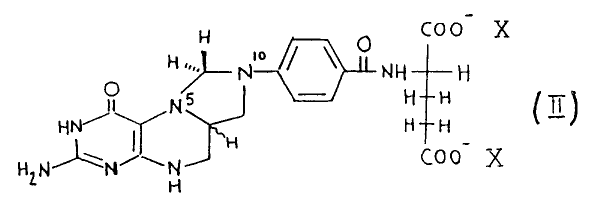 in der Form des (6RS)-Diastereoisomerengemisches oder in der Form der einzelnen (6S)- oder (6R)-Diastereoisomeren,
worin
X ein Alkalimetallkation oder ein Ammoniumion bedeutet,
in wässriger Lösung mit wenigstens einem Alkalimetallborhydrid,
gefolgt von der Hinzugabe wenigstens eines Aequivalentes eines Erdalkalimetallsalzes und der Abtrennung des entstandenen Festkörpers, welcher die genannten Salze der Formel I enthält,
dadurch gekennzeichnet, dass man den genannten Festkörper in Wasser löst und dazu wenigstens eine Verbindung A hinzugibt, welche insbesondere für die Komplexierung von Bor enthaltenden Verbindungen B geeignet ist,
dann das so hergestellte Gemisch C einengt, wobei eine Kristallisation eintritt, und die entstandenen, reinen Kristalle der Formel I mittels Filtration oder Zentrifugation isoliert.

2. Verfahren nach Anspruch 1, dadurch gekennzeichnet, dass die Verbindung A ausgewählt ist aus der Gruppe, bestehend aus Ethylen-diamintetraessigsäure, abgekürzt mit EDTA, und deren Salze und Hydrate, insbesondere das di-Natriumsalz, Nitrilotriessigsäure, 1,2-Diaminocyclohexan-N,N,N',N'-tetraessigsäure und polymer gebundenen Komplexierungsmitteln, wie etwa Chelosolve, wobei EDTA und deren di-Natriumsalz bevorzugt sind.

3. Verfahren nach einem der Ansprüche 1 bis 2, dadurch gekennzeichnet, dass man von 5 bis 30 Milliequivalente, abgekürzt mit meq, an Verbindung A pro Mol an zu reinigender Verbindung der Formel I verwendet.

4. Verfahren nach einem der Ansprüche 1 bis 3, dadurch gekennzeichnet, dass man das Gemisch C auf einen Drittel bis einen Viertel seines ursprünglichen Volumens einengt, insbesondere unter reduziertem Druck bei einer Temperatur von 45°C bis 50°C.

5. Verfahren nach einem der Ansprüche 1 bis 4, dadurch gekennzeichnet, dass das verwendete Alkalimetallborhydrid Natriumborhydrid ist.

6. Verfahren nach einem der Ansprüche 1 bis 5, dadurch gekennzeichnet, dass man die genannten Festkörper unter einer Inertgasatmosphäre bei einer Temperatur von etwa 70°C in Wasser, vorzugsweise deionisiertes Wasser, löst.

7. Verwendung der Erdalkalimetallsalze von N(5)-Methyl-5,6,7,8-tetrahydrofolsäure der Formel I 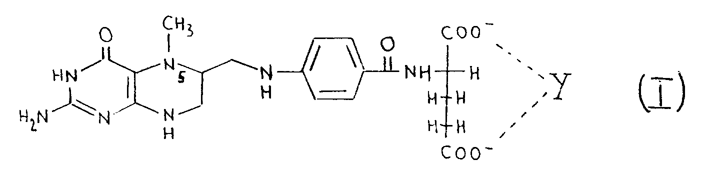 in der Form des (6RS)-Diastereoisomerengemisches oder in der Form der einzelnen (6S)-oder (6R)-Diastereoisomeren,
worin
Y ein Erdalkalimetallkation, insbesondere Ca²⁺ oder Mg²⁺, bedeutet,
hergestellt gemäss dem Verfahren nach einem der Ansprüche 1 bis 6,
einzeln oder in beliebiger Kombination als wenigstens eine aktive Komponente in einem Arzneimittel für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen.

8. Verwendung nach Anspruch 7, dadurch gekennzeichnet, dass die Salze der Formel I in einer Menge von 1 mg bis 500 mg, insbesondere von 5 mg bis 150 mg, pro Dosiseinheit im Arzneimittel vorhanden sind, und wobei das Arzneimittel vorzugsweise in Form eines parenteralen und/oder oralen, pharmazeutischen Präparates vorliegt.

9. Verwendung nach einem der Ansprüche 7 bis 8, dadurch gekennzeichnet, dass das Arzneimittel noch wenigstens einen Stabilisator, beispielsweise Ascorbinsäure, insbesondere in einer Menge von 1 bis 25 Gew.-%, bezogen auf die Menge an vorhandener aktiver Verbindung, enthält.
